Europäisches Patentamt

European Patent Office

Office européen des brevets

(11) Veröffentlichungsnummer: **0 528 437 A1**

# EUROPÄISCHE PATENTANMELDUNG

(21) Anmeldenummer: **92114309.5**

(22) Anmeldetag: **21.08.92**

(51) Int. Cl.5: **G01F 1/704**, **A61M 5/168**

(30) Priorität: **21.08.91 DE 4127675**

(43) Veröffentlichungstag der Anmeldung:
**24.02.93 Patentblatt 93/08**

(84) Benannte Vertragsstaaten:
**AT BE CH DE DK ES FR GB IT LI NL PT SE**

(71) Anmelder: **B. BRAUN MELSUNGEN AG**
**Postfach 120**
**W-3508 Melsungen(DE)**

(72) Erfinder: **Heitmeier, Rolf, Dipl.-Ing.**
**Goethestrasse 8**
**W-3507 Baunatal(DE)**
Erfinder: **Wolfram, Berthold**
**Sonnenhang 25**
**W-3508 Melsungen(DE)**

Erfinder: **von der Haar, Friedrich, Prof. Dr. rer. nat.**
**Tilsiterstrasse 2**
**W-3508 Melsungen(DE)**
Erfinder: **Just, Hans-Jürgen, Dr. rer. nat. Dipl.-Chem.**
**Karower-Chaussee 73**
**W-1115 Berlin(DE)**
Erfinder: **Sichting, Thomas, Dipl.-Ing.**
**Wiesengrund 13 b**
**W-1409 Mühlenbeck(DE)**

(74) Vertreter: **Klingseisen, Franz, Dipl.-Ing.**
**Dr. F. Zumstein Dipl.-Ing. F. Klingseisen**
**Bräuhausstrasse 4**
**W-8000 München 2 (DE)**

(54) **Verfahren und Vorrichtung zum Überwachen und Messen der Strömung eines Fluids in einer Leitung, insbesondere für Infusionssysteme.**

(57) Bei einem Verfahren zum Überwachen der Strömung eines Fluids in einer Leitung, insbesondere für Infusionssysteme, wobei dem Fluid Wärmeimpulse zugeführt und in einem Abstand davon stromab diese Wärmeimpulse abgetastet werden, wird zur Erzielung einer hohen Empfindlichkeit und eines schnellen Ansprechvermögens bei der Strömungsüberwachung die Heizzeit beim Aufbringen der Wärmeimpulse der Strömungsgeschwindigkeit des Fluids automatisch oder manuell angepaßt. Es wird eine Vorrichtung zum Überwachen und Messen der Fluidströmung vorgesehen, bei dem wenigstens ein Heizelement (2) und ein Temperatursensor (8) auf dem Außenumfang der Leitung (1) angeordnet sind, um die Strömung des Fluids nicht zu beeinflußen und die Handhabung der Strömungsüberwachung zu vereinfachen.

Fig. 1

Die Erfindung betrifft ein Verfahren nach dem Oberbegriff des Anspruchs 1 sowie eine Vorrichtung nach dem Oberbegriff des Anspruchs 8.

Die Überwachung der Langzeit-Medikamentenversorgung von Patienten nimmt bei abnehmender Halbwertszeit der Medikamente an Bedeutung zu. Im Vordergrund steht hierbei die Aufrechterhaltung von kleinsten Volumenströmen ( z.B. 1 ml/h) und deren Kontrolle bei Infusionsgeräten.

Um lange Reaktionszeiten bis zum Feststellen einer beispielsweise durch eine Schlauchokklusion hervorgerufenen Unterbrechung des Volumenstromes bei einem Infusionsgerät und damit eine Patientengefährdung zu vermeiden, ist es aus EP 0 405 148 A1 bekannt, einen Thermo-Strömungssensor mit einem mit der strömenden, wärmeabführenden Flüssigkeit in thermischem Kontakt stehenden Heizelement vorzusehen, wobei eine auf die Wärmeabfuhr von dem Heizelement ansprechende Auswertschaltung vorgesehen ist, die bei nichtströmender Flüssigkeit oder bei Anwesenheit wärmeisolierender Gasblasen in der Umgebung des Heizelementes ein Ausgangssignal zur Betätigung einer Alarmeinrichtung erzeugt. Hierbei wird ein Heizdrahtwiderstandselement in den Infusionsschlauch eingebracht, so daß der Strömungsweg unmittelbar beeinflußt wird. Zudem ist bei diesem bekannten Aufbau ein spezieller steriler Einmalartikel für den Strömungssensor erforderlich.

Zur Vermeidung des unmittelbaren Kontaktes des Heizelementes bzw. Sensors mit der zu überwachenden Flüssigkeitsströmung ist es aus der US-PS 4 813 280 bei einer an eine Schlauchleitung anschließbaren Meßzelle bekannt, in einem Abstand voneinander radiale Bohrungen in den rohrförmigen Körper der Meßzelle einzubringen und den Rohrkörper mit einer flexiblen Gummihülle zu überziehen, so daß durch die eine radiale Bohrung ein Heizelement und durch die andere ein Sensor eingedrückt werden kann, wobei sich die Gummihülle entsprechend dehnt, so daß Heiz- und Sensorelement in den Strömungsweg in dem rohrförmigen Körper vorstehen. Hierdurch wird die Strömung beeinflußt, wobei an der Querschnittsverengung die Strömungsgeschwindigkeit erhöht wird. Durch die in der Leitung auftretenden unterschiedlichen Strömungsgeschwindigkeiten kommt es zur Ausgasung und Anlagerung von Gasblasen. Dies ist vor allem bei einem medizinischen Infusionsgerät von Nachteil.

Durch das Heizelement werden bei dieser bekannten Meßzelle Wärmeimpulse in das zu überwachende Fluid eingebracht, die durch das Sensorelement abgetastet werden. Die Zeit zwischen aufeinanderfolgenden Wärmeimpulsen ergibt die Strömungsgeschwindigkeit des Fluids durch die Meßzelle. Für sehr geringe Volumenströme, wie sie bei neueren Infusionsgeräten gefordert werden, ist diese Art der Strömungsmessung zu ungenau. Die Meßgröße wird durch Störfaktoren überlagert und die Ansprechzeit bis zum Feststellen einer Unterbrechung des Volumenstroms entspricht nicht den Erfordernissen.

Der Erfindung liegt die Aufgabe zugrunde, ein Verfahren der eingangs angegebenen Art so auszubilden, daß man eine hohe Empfindlichkeit und ein schnelles Ansprechvermögen bei der Strömungsüberwachung erhält. Ferner soll eine Vorrichtung der eingangs angegebenen Art so ausgebildet werden, daß die Strömung des Fluids nicht beeinflußt und die Handhabung bei der Strömungsüberwachung wesentlich vereinfacht wird.

Diese Aufgabe wird erfindungsgemäß durch die Merkmale im Kennzeichen des Anspruchs 1 bzw. des Anspruchs 8 gelöst.

Dadurch, daß nach Anspruch 1 die Heizzeit der dem Fluid zugeführten Wärmeimpulse in Abhängigkeit von der Strömungsgeschwindigkeit des Fluids eingestellt wird, kann der Einfluß von Fluidtemperaturschwankungen auf das Sensorsignal sowie der Einfluß anderer Störfaktoren minimiert werden. Man erhält eine hohe Empfindlichkeit und kurze Ansprechzeit, weil jeweils nur die für eine bestimmte Strömungsgeschwindigkeit minimale Heizzeit eingestellt werden kann, die zu einem deutlichen, vorzugsweise im wesentlichen maximalen Sensorsignal führt, an dessen Veränderung kurzfristig eine Änderung der Strömungsgeschwindigkeit bzw. eine Unterbrechung des Volumenstroms festgestellt werden kann.

Durch die Anordnung des Heizelementes und des Temperatursensors auf der Außenseite der Schlauchleitung nach Anspruch 8 entfällt ein in die Schlauchleitung einsetzbares Element. Auch wird hierbei die Schlauchleitung selbst nicht deformiert, so daß die Strömung in der Schlauchleitung in keiner Weise durch diese Überwachungsvorrichtung beeinflußt wird. Die Handhabung ist dadurch sehr einfach, daß die Überwachungsvorrichtung an jeder beliebigen Stelle an der Schlauchleitung angesetzt werden kann.

Vorteilhafte Ausgestaltungen der Erfindung sind in der nachfolgenden Beschreibung und in den weiteren Ansprüchen angegeben.

Eine beispielsweise Ausführungsform der Erfindung wird nachfolgend an Hand der Zeichnung näher erläutert. Es zeigen

Fig. 1     einen schematischen Querschnitt durch eine erfindungsgemäße Vorrichtung,

Fig. 2     in einem Diagramm den Einfluß der Strömungsgeschwindigkeit auf das Sensorsignal,

Fig. 3     in einem Diagramm die minimale Heizzeit als Funktion des Volumenstroms, und

Fig. 4 Beispiele der unterschiedlichen Impulshöhe des Sensorsignals in Abhängigkeit von der Strömungsgeschwindigkeit.

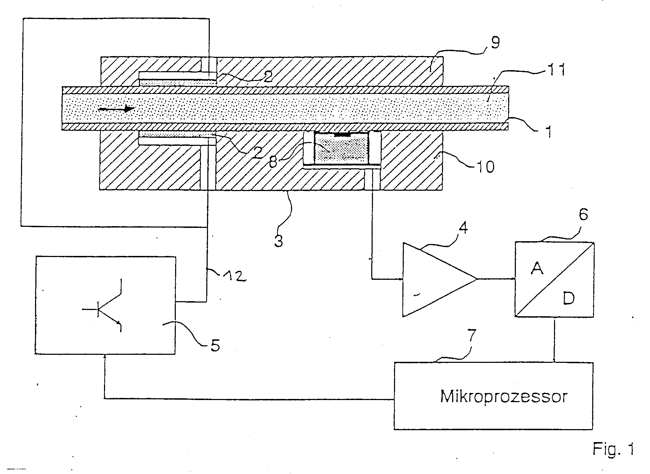

In Fig. 1 ist mit 1 eine Schlauchleitung bezeichnet, die von einem zu überwachenden Fluid 11 in Pfeilrichtung durchströmt wird. Auf dem Außenumfang bzw. der Außenseite der Schlauchleitung 1 ist dicht anliegend ein flächiges Heizelement 2 angeordnet, das über eine Anschlußleitung 12 von einem Schaltteil 5 mit Heizstrom versorgt wird, das von einem Mikroprozessor 7 gesteuert wird. Das Heizelement 2 kann sich über einen Teil oder über den gesamten Umfang der Schlauchleitung 1 erstrecken. Bei dem dargestellten Ausführungsbeispiel sind zwei einander diametral gegenüberliegende Heizelemente 2 vorgesehen. Die Heizelemente haben eine sehr geringe Masse, und sie sind vorzugsweise als Widerstandselemente in SMD-Technik oder als dünne Heizfolien ausgebildet.

In einem bestimmten Abstand stromab des oder der Heizelemente 2 ist am Umfang der Schlauchleitung 1 ein Temperatursensor 8 angeordnet, der vorzugsweise als Infrarotstrahlungsdetektor ausgebildet ist. Auf diese Weise wird eine trägheitslose Erfassung von Temperaturänderungen in dem zu überwachenden Fluid 11 erreicht. Auf Grund der hohen Empfindlichkeit des Temperatursensors 8 können die durch die Heizelemente 2 erzeugten, pulsförmigen Temperaturveränderungen kleiner als 2 Kelvin sein. Man kann somit mit einer geringen Heizleitung arbeiten. Der Temperatursensor 8 ist über einen Verstärker 4 und einen Analog/Digital-Wandler 6 mit dem Mikroprozessor 7 verbunden.

Als Material für die Schlauchleitung 1 wird vorzugsweise ein im Infrarotbereich transparentes Material verwendet, jedoch kann auch, wie Versuche gezeigt haben, nicht infrarotdurchlässiges Material für die Schlauchleitung verwendet werden. Zweckmäßigerweise wird für die Schlauchleitung PVC oder Polyäthylen vorgesehen. Heizelement 2 und Temperatursensor 8 sind derart auf der Außenfläche der Schlauchleitung 1 angeordnet, daß diese nicht deformiert wird, so daß die Strömung in der Schlauchleitung in keiner Weise beeinflußt wird. Die Sterilität der durch die Schlauchleitung strömenden, zu infundierenden Flüssigkeit wird dadurch gewährleistet.

Das Heizelement 2 ist zusammen mit dem Temperatursensor 8 in einem gemeinsamen Gehäuse 3 eingebaut, das eine für die Aufnahme der Schlauchleitung 1 dimensionierte Führungsnut aufweist. Beispielsweise kann diese Führungsnut etwa U-förmig auf einer Seite des Gehäuses 3 ausgebildet sein, so daß die eingelegte Schlauchleitung 1 auf einem Halbkreis am Nutgrund anliegt. Auf diesem Halbkreis ist das Heizelement 2 und der Temperatursensor 8 angeordnet. Vorzugsweise wird das Gehäuse 3 geteilt ausgebildet, wobei ein schematisch angedeuteter Deckel 9 und ein Trägerteil 10 vorgesehen wird. Auf diese Weise kann die Schlauchleitung 1 vollständig vom Gehäuse 3 umschlossen werden und es können zwei diametral gegenüberliegende Heizelemente 2 auf der Außenseite der Schlauchleitung 1 angelegt werden, wie Fig. 1 zeigt.

Dieser Aufbau mit Heizelement und Temperatursensor in einem gemeinsamen Gehäuse 3 erlaubt es, die Messung und Überwachung an jeder beliebigen Schlauchstelle vorzunehmen. Die Schlauchleitung kann problemlos in die Führungsnut des Gehäuses eingelegt und wieder entfernt werden.

Weiterhin ist das Gehäuse 3 so ausgebildet, daß die Meßstrecke zwischen Heizelement 2 und Temperatursensor 8 gegenüber kurzfristig auftretenden Umgebungseinflüssen wie Temperaturschwankungen abgeschirmt wird. Dies kann durch eine geeignete Materialwahl für das Gehäuse 3 und durch ausreichende Masse des Gehäuses erfolgen.

Wie Fig. 1 zeigt, sind die Heizelemente 2 und der Temperatursensor 8 derart im Gehäuse 3 angeordnet, daß sie mit ihren sensitiven Flächen mit der Oberfläche der Führungsnut im Gehäuse fluchten, so daß sich bei eingelegter Schlauchleitung 1 eine dichte Anlage an deren Außenseite ergibt.

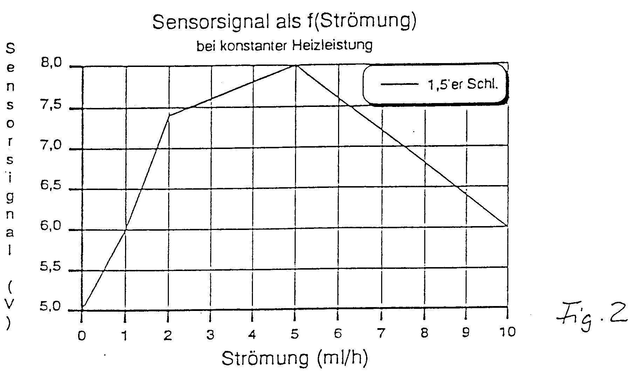

Wird den Heizelementen 2 eine geringe Heizleistung von ca. 250 mW zugeführt, so wandelt der hochempfindliche Strahlungssensor 8 den sich ausbildenden Temperaturgradienden zwischen Heizelement 2 und Sensor 8 in ein elektrisches Signal um. Neben der Wärmeleitung durch das Gehäuse und längs der Schlauchoberfläche findet eine Wärmeleitung durch das Fluid statt. Die sich im Fluid ausbildende Wärmeverteilung wird durch die Strömung derart beeinflußt, daß an der Stelle des Temperatursensors 8 ein Zuwachs der Fluidtemperatur als Funktion der Strömung detektiert werden kann. Dieser Zusammenhang ist in Fig. 2 dargestellt.

Bei einer vorgegebenen Schlauchleitung 1 und konstanter Heizleistung an den Heizelementen 2 in Form zeitgleicher Heizimpulse erhält man in Abhängigkeit vom Volumenstrom ml/h ein Sensorsignal V als Funktion des Volumen-stroms, wobei das Sensorsignal V bis zu einem Volumenstrom von etwa 5 ml/h ansteigt und danach wieder abfällt. Hierbei ergibt sich ein eingeschränkter Meßbereich bei kleineren Volumenströmen.

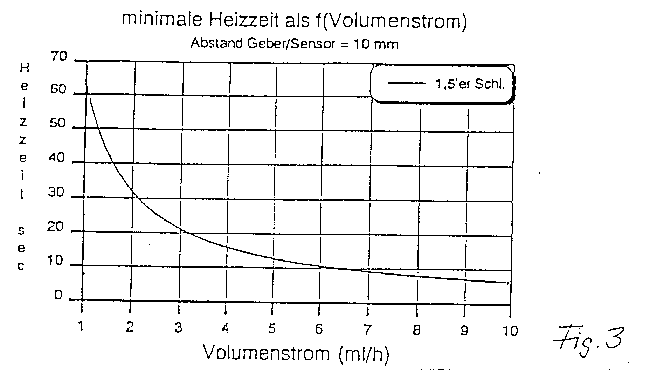

Dieser eingeschränkte Meßbereich sowie der Einfluß von Temperaturschwankungen des Fluids auf das Ausgangssignal des Temperatursensors 8 werden dadurch aufgehoben, daß die Heizzeit t in Abhängigkeit von der Strömungsgeschwindigkeit bzw. vom Volumenstrom ml/h verändert bzw. eingestellt wird. Fig. 3 zeigt die notwendige bzw. minimale Heizzeit t in sec in Abhängigkeit vom Volumenstrom ml/h bei einem vorgegebenen Abstand von Heizelement und Temperatursensor von 10 mm unter Verwendung der gleichen Schlauchleitung, die auch dem Diagramm nach Fig. 2 zugrundeliegt. Danach wird bei geringem Volumenstrom die Heizzeit erhöht und bei größerem Volumenstrom verringert. Wird die Heizzeit dem jeweiligen Volumenstrom in dieser Weise automatisch oder manuell angepaßt, so erhält man eine Erweiterung des Meßbereichs zu kleineren Volumenströmen hin von beispielsweise bis zu 0,5 ml/h und eine Optimierung der Reaktionszeit des Systems.

Vorzugsweise wird die Heizzeit t wie folgt festgelegt:

$$t > = A \cdot s/Q$$

wobei A der Strömungsquerschnitt der Schlauchleitung 1, s der Abstand zwischen Heizelement 2 und Temperatursensor 8 und Q der vorgegebene Volumenstrom ist.

Die Pausenzeiten zwischen den Wärmeimpulsen bzw. Heizzeiten t werden im wesentlichen gleich der oder größer als die Heizzeit ausgelegt.

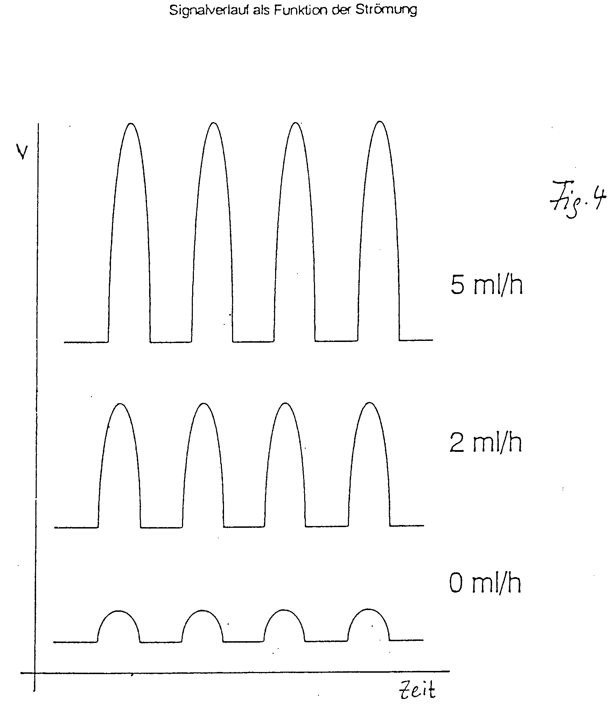

Für eine vorgegebene Überwachungsvorrichtung und eine vorgegebene Schlauchleitung wird der Volumenstrom durch eine nicht dargestellte Infusionspumpe vorgegeben. Unter Verwendung der oben angegebenen Beziehung zwischen Heizzeit und vorgegebenen Vorrichtungsparametern wird die minimale Heizzeit nach Fig. 3 in Abhängigkeit vom Volumenstrom festgelegt. Entsprechend dieser Beziehung nach Fig. 3 wird einem an der Pumpe eingestellten Volumenstrom eine bestimmte Heizzeit zugeordnet, innerhalb der ein maximales Sensorsignal V erreicht wird. Wird an der Pumpe ein anderer Volumenstrom eingestellt, so wird die Heizzeit entsprechend Fig. 3 verändert. Dadurch, daß man einem vorgegebenen Volumenstrom eine bestimmte Heizzeit zuordnet, erhält man eine vorgegebene Impulshöhe des Sensorsignals V über der Zeit, wie dies Fig. 4 nur schematisch bei verschiedenen Volumenströmen zeigt, während die Zuordnung der Heizzeit zu bestimmten Volumenströmen in Fig. 3 angegeben ist, aus der sich z.B. bei 1 ml/h eine Heizzeit t = 60 s und bei 5 ml/h eine Heizzeit t = 13 s ergibt. Damit kann an der Impulshöhe die tatsächlich vorhandene Strömungsgeschwindigkeit abgeleitet werden. Verändert sich die Impulshöhe des Sensorsignals, so kann daraus eine entsprechende Änderung der Strömungsgeschwindigkeit abgeleitet werden. Auf diese Weise wird durch die Anpassung der Heizzeit an die Strömungsgeschwindigkeit des Fluids bei vorgegebenen Vorrichtungsparametern eine optimale Empfindlichkeit bei der Überwachung der Fluidströmung erreicht.

Bei dem beschriebenen Verfahren wird der Temperaturverlauf des Fluids über der Zeit ermittelt. Dieses Temperaturprofil, das als Signal am Sensor 8 zur Verfügung steht, wird bewertet. Um ein meßbares Temperaturprofil zu erhalten, wird die periodische Einschaltdauer der Heizung bzw. die Heizzeit der Strömungsgeschwindigkeit angepaßt. Dabei wird die Amplituden- bzw. Impulshöhe des ermittelten Temperaturprofils bewertet.

Das bei 4 verstärkte Signal vom Temperatursensor 8 hat, wie dies Fig. 4 zeigt, beispielsweise bei einem Volumenstrom von 5 ml/h eine größere Impulshöhe, bei 2 ml/h eine geringere Impulshöhe und bei Strömungsstillstand 0 ml/h eine minimale Impulshöhe, die sich durch Wärmeleitung durch das Gehäuse und die Schlauchleitung sowie das stillstehende Fluid ergibt. Durch diese Signalform kann nach erfolgter Digitalisierung bei 6 der nachgeschaltete Mikroprozessor 7 Temperaturschwankungen des Fluids wie auch andere Störungen des vom Temperatursensor 8 kommenden Signals durch Plausibilitätskontrollen herausfiltern, da das Sensorsignal aus einem Temperaturoffset ( durch unterschiedliche Fluidtemperaturen ) und einem Temperaturwechselanteil ( durch die Heizimpulse ) besteht. Eine geeignete Programmlogik des Mikroprozessors (z.B. Herausfiltern der Minimal- und Maximalwerte) ist aufgrund der provozierten Amplitudenschwankungen des Signals in der Lage, das Nutzsignal von den Störsignalen zu trennen und anschließend in seiner Impulshöhe zu bewerten. Störeinflüsse werden dadurch weitestgehend unterdrückt.

Wird in die Programmlogik des Mikroprozessors 7 eine minimale zulässige Impulshöhe eingeführt, so kann bei deren Unterschreiten ein Alarm ausgelöst werden. Hierdurch wird eine einfache Überwachung der Strömung erreicht.

Um eine Unterbrechung der Strömung möglichst kurzfristig zu erkennen, wie dies bei der medizinischen Anwendung von besonderer Bedeutung ist, bei der das Fluid ein Medikament enthält, kann nach einem weiteren Aspekt der Erfindung die Heizzeit der tatsächlichen Strömungsgeschwindigkeit kontinuierlich angepaßt werden. Der Mikroprozessor 7 bewertet hierzu den Signalverlauf am Temperatursensor 8. Es wird dabei die Zeitdifferenz zwischen Maximalwert des Sensorsignals und dem Abschaltzeitpunkt der Heizung gebildet. Diese Differenz wird minimiert, so daß dann, wenn der Maximalwert vor dem Ende der Heizzeit auftritt, die Heizzeit verkürzt wird, und dann, wenn der Maximalwert nach dem Ende der Heizzeit erreicht wird, die Heizzeit verlängert wird.

Auf diese Weise wird für jeden Volumenstrom im Bereich zwischen 0,1 und 10 ml/h die optimale Impulsfolge an den Heizelementen 2 bezüglich Empfindlichkeit und kürzester Reaktionsgeschwindigkeit gefunden.

Gegenüber diesem automatischen Verfahren, bei dem die Strömungsgeschwindigkeit des Fluids nicht vorgegeben werden muß, ist es sinnvoll, nicht auf jede kurzfristige Schwankung der Strömungsgeschwindigkeit die Heizzeit erneut zu adaptieren. Es ist auch möglich, am Sensorsystem den zu überwachenden Volumenstrom in seinem Nennwert einzustellen, worauf das System so ausgelegt werden kann, daß es danach entsprechend dem in Fig. 3 wiedergegebenen Zusammenhang die erforderliche Heizzeit selbständig einstellt.

Ein weiterer Vorteil liegt darin, daß durch entsprechende Dimensionierung der Heizleistung oder des Abstandes zwischen Heizelement und Temperatursensor die Funktionsfähigkeit der Meßstrecke im Betrieb überwacht werden kann, da eine minimale Impulshöhe auch bei Strömungsstillstand erreicht wird, wie Fig. 4 zeigt. Dies ist besonders für sicherheitsrelevante Anwendungen von Bedeutung. Der Ausfall der Überwachungsvorrichtung wird durch gänzlich fehlende Impulse vom Mikroprozessor 7 erkannt. Gleiches gilt für die Feststellung von größeren Gasblasen in der Fluidströmung, da auch durch Gasblasen in der Strömung die Impulshöhe des Sensorsignals merklich gedämpft wird.

Das beschriebene Verfahren kann sowohl zum Überwachen als auch zum Messen der Strömungsgeschwindigkeit des Fluids eingesetzt werden.

**Patentansprüche**

1. Verfahren zum Überwachen der Strömung eines Fluids in einer Leitung, insbesondere für Infusionssysteme, wobei das Fluid an einer Stelle der Leitung durch eine Heizung (2) intermittierend beheizt und stromab in einem Abstand davon die Temperatur des Fluids durch einen Temperatursensor (8) erfaßt wird,
   **dadurch gekennzeichnet,**
   daß die Heizzeit (t) der einzelnen Heizperioden in Abhängigkeit von der Strömungsgeschwindigkeit des Fluids automatisch oder manuell eingestellt wird.

2. Verfahren nach Anspruch 1,
   dadurch gekennzeichnet,
   daß die Heizzeit (t) bei vorgegebenem Volumenstrom (Q) so festgelegt wird, daß
   $t \geq A \cdot s/Q$,
   wobei A der Strömungsquerschnitt und s der Abstand zwischen Heizung (2) und Temperatursensor (8) ist.

3. Verfahren nach den Ansprüchen 1 und 2,
   dadurch gekennzeichnet,
   daß die Pausenzeiten zwischen aufeinanderfolgenden Heizperioden annähernd gleich der oder größer als die Heizzeit (t) ist.

4. Verfahren zum Überwachen der Strömung eines Fluids in einer Leitung, insbesondere für Infusionssysteme, wobei das Fluid an einer Stelle der Leitung durch eine Heizung (2) intermittierend beheizt und stromab in einem Abstand davon die Temperatur des Fluids durch einen Temperatursensor (8) erfaßt wird,
   dadurch gekennzeichnet,
   daß die Zeitdifferenz zwischen dem Zeitpunkt, bei dem an dem Temperatursensor (8) ein maximales Signal auftritt, und dem Abschaltzeitpunkt der Heizung (2) gebildet wird und daß diese Zeitdifferenz minimiert wird.

5. Verfahren nach Anspruch 4,
   dadurch gekennzeichnet,
   daß bei Auftreten des Maximalwerts des Sensorsignals vor dem Ende der Heizphase die Heizzeit verkürzt wird und bei Auftreten des Maximalwerts nach dem Ende der Heizphase die Heizzeit verlängert wird.

6. Verfahren nach den vorhergehenden Anspürchen,
   dadurch gekennzeichnet,
   daß eine minimale zulässige Impulshöhe des Sensorsignals festgelegt und bei Unterschreiten dieser Impulshöhe ein Alarm ausgelöst wird.

7. Verfahren nach den vorhergehenden Ansprüchen,
   dadurch gekennzeichnet,
   daß Temperaturwechsel aus dem Sensorsignal herausgefiltert und deren Amplitudenänderungen bewertet werden.

8. Vorrichtung zum Überwachen und Messen der Strömung eines Fluids in einer Leitung, insbesondere für Infusionsgeräte, mit einem Heizelement und einem stromab von diesem angeordneten Temperatursensor,
   dadurch gekennzeichnet,
   daß wenigstens ein Heizelement (2) und der Temperatursensor (8) auf dem Außenumfang der Leitung (1) angeordnet sind.

9. Vorrichtung nach Anspruch 8,
   dadurch gekennzeichnet,
   daß das Heizelement (2) als Widerstandselement in SMD-Technik oder als dünne Heizfolie ausgebildet ist.

10. Vorrichtung nach Anspruch 8,
    dadurch gekennnzeichnet,

daß der Temperatursensor (8) als Infrarotstrahlungssensor ausgebildet ist und das Material der Leitung (1) ein im Infrarotbereich transparentes Material ist.

11. Vorrichtung nach den Ansprüchen 8 bis 10, dadurch gekennzeichnet, daß das Heizelement (2) und der Temperatursensor (8) in einem gemeinsamen Gehäuse (3) angeordnet sind, das mit einer Nut zur Aufnahme der Schlauchleitung (1) versehen ist, wobei im Anlagebereich der Schlauchleitung an der Nutwandung das Heizelement und der Temperatursensor angeordnet sind.

12. Vorrichtung nach Anspruch 11, dadurch gekennzeichnet, daß das Gehäuse (3) geteilt ausgebildet und die Schlauchleitung (1) im geschlossenen Zustand des Gehäuses auf dem gesamten Umfang von diesem umschlossen ist.

13. Vorrichtung nach den Ansprüchen 11 und 12, dadurch gekennzeichnet, daß das Gehäuse (3) zur Abschirmung gegen Außentemperaturschwankungen wärmeisolierend ausgebildet ist.

Fig. 1

Sensorsignal als f(Strömung)
bei konstanter Heizleistung

Fig. 2

## minimale Heizzeit als f(Volumenstrom)

### Abstand Geber/Sensor = 10 mm

Fig. 3

Signalverlauf als Funktion der Strömung

Fig. 4

5 ml/h

2 ml/h

0 ml/h

V

Zeit

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-2 157 850 (THE DISTILLERS COMPANY) <br> * Zusammenfassung * <br> --- | 1-8 | G01F1/704 <br> A61M5/168 |
| X | EP-A-0 386 670 (IVAC CORP.) <br> * Spalte 15, Zeile 14 - Zeile 22 * <br> * Spalte 16, Zeile 43 - Spalte 17, Zeile 19; Abbildungen * <br> --- | 1,8 <br> 10-13 | |
| X | EP-A-0 289 361 (MEDISTRON LTD) <br> * Spalte 8, Zeile 36 - Spalte 9, Zeile 11; Abbildungen 1,2 * <br> --- | 8-10 | |
| X | US-A-4 384 578 (WINKLER) <br> * das ganze Dokument * <br> --- | 8,9 <br> 11-13 | |
| A | EP-A-0 072 044 (DOW CHEMICAL CO.) <br> * Zusammenfassung * <br> ----- | 1-13 | |

RECHERCHIERTE SACHGEBIETE (Int. Cl.5)

G01F
A61M

Der vorliegende Recherchenbericht wurde für alle Patentansprüche erstellt

| Recherchenort | Abschlußdatum der Recherche | Prüfer |
|---|---|---|
| DEN HAAG | 25 NOVEMBER 1992 | CLARKSON P. |